# EUROPEAN PATENT APPLICATION

(11) **EP 4 725 505 A1**
(43) Date of publication of application: **15.04.2026**
(21) Application number: 24835817.8
(22) Date of filing: 31.05.2024
(51) Int. Cl.: A61K 45/00, A61K 31/121, A61K 31/122, A61K 31/365, A61K 31/4155, A61K 31/423, A61K 31/60, A61P 17/00, A61P 17/16, A61P 43/00, C12N 9/99, C12Q 1/02, C12Q 1/6883, G01N 33/15, G01N 33/50

(54) **PROPHYLACTIC AND/OR THERAPEUTIC AGENT FOR SKIN DISORDER ASSOCIATED WITH HISTONE DEACETYLASE 4 ABNORMALITY**

(30) Priority: 03.07.2023 JP 2023109275
(71) Applicant: National University Corporation Kobe University, Kobe-shi, Hyogo 657-8501 (JP)
(72) Inventor: FUKUMOTO, Takeshi, Kobe-shi, Hyogo 657-8501 (JP); NISHIGORI, Chikako, Kobe-shi, Hyogo 657-8501 (JP); AOI, Takashi, Kobe-shi, Hyogo 657-8501 (JP); AOI, Michiyo, Kobe-shi, Hyogo 657-8501 (JP); TAKEMORI, Chihiro, Kobe-shi, Hyogo 657-8501 (JP)
(74) Representative: Arnold & Siedsma
(86) International application number: PCT/JP2024/020054
(87) International publication number: WO 2025/009296

(57) **Abstract**

Provided is a prophylactic and/or therapeutic agent for a skin disorder associated with a histone deacetylase 4 abnormality. The prophylactic and/or therapeutic agent for a skin disorder associated with a histone deacetylase 4 abnormality includes a histone acetyltransferase inhibitor as an active ingredient. The prophylactic and/or therapeutic agent can prevent and/or treat the skin disorder associated with the histone deacetylase 4 abnormality.

## Description

### Technical Field

The present invention relates to a prophylactic and/or therapeutic agent for a skin disorder associated with a histone deacetylase 4 abnormality.

The present application claims the priority of Japanese Patent Application No. 2023-109275, which is incorporated herein by reference.

### Background Art

A pigment cell (melanocyte) is a cell that is derived from a neural crest in its developmental process and produces a melanin. The melanocyte is present mainly in the basal layer of epidermis in skin, and supplies a melanin to a peripheral keratinocyte. Although the melanocyte is responsible for the defense of the skin from UV light through melanin production, the melanocyte causes DNA damage owing to the UV light.

In xeroderma pigmentosum (hereinafter also simply referred to as "(XP)"), a target organ in which its symptom is expressed most strongly is skin, and in particular, dyschromatosis is inevitable. Accordingly, the analysis of the UV response of a melanocyte in a patient is most important in the pathological elucidation of XP. XP is a hereditary photosensitive disease, which is inherited in an autosomal recessive manner, and is a hereditary DNA repair deficiency disorder that causes severe photosensitivity and a severe neurological symptom. The skin of an XP patient is congenitally deficient in ability to repair DNA damage caused by UV light, and hence severe photosensitivity to sunlight or UV light (e.g., abnormal sunburn, dyschromatosis, or the skin cancer of an exposed portion) occurs to various extents depending on the disease type of XP. Accordingly, a light-shielding environment needs to be formed in the whole living space, and hence the QOL of the XP patient and his or her family remarkably reduces. When appropriate light shielding is neglected, a severe photosensitive symptom or severe freckle-like dyschromatosis progresses to develop skin cancer at a high frequency despite the fact that the patient is young. Further, one half or more of XP patients involve progressive brain-wasting and neurodegenerative diseases of unknown origin, and the severity of such disease affects the prognosis of each of the patients. Accordingly, the elucidation of the pathology of XP and the establishment of a therapeutic drug therefor have been strongly desired, and the establishment of the therapeutic drug is an urgent need for XP patients in the world.

XP is classified into the following eight disease types: a group A to a group G (genetically complementary groups) that are genetically different from each other, and a variant (V) type. It has been said that the group A (xeroderma pigmentosum group A: XP-A), which is such a type that both of a skin symptom and a neurological symptom are most severe, accounts for about 55% of XP in Japan, and the V type, in which only the skin symptom occurs, accounts for about 25% thereof. In each of XP-A patients often found in Japanese people, a photosensitive symptom is severe, and hence a severe sunburn symptom starts to appear immediately after his or her birth, and markedly accelerated photoaging is observed even when the amount of UV light is low. That is, skin symptoms, such as a severe sunburn reaction, freckle-like pigmented maculae that have various sizes and various color tones, and the skin cancer of an exposed portion in a juvenile, are observed. However, the pathology of XP-A has not been elucidated yet. In addition, in each of the XP-A patients, a neurological symptom, such as hearing loss, an indistinct language, or an inability to keep his or her body balance, manifests itself when the patient is about 6 years old, and a neurological symptom, such as a liability to fall, gradually starts to appear when the patient is more than 10 years old. Along with the progress of the hearing loss or intellectual disability of the patient, the language function thereof reduces.

Research in a melanocyte is important in the advance of the pathological elucidation of XP because a target organ in which its symptom is expressed most strongly is skin, and in particular, dyschromatosis is inevitable. However, the pathology of XP has been analyzed by using a fibroblast (Non Patent Literatures 1 and 2) because it is difficult to isolate and culture the melanocyte from the skin tissue of an XP patient. Meanwhile, it is important to perform the research through use of the melanocyte in XP (Non Patent Literatures 3 to 5) because the melanocyte and the fibroblast are different from each other in resistance to UV light and reaction to DNA damage. However, there has heretofore been no report on the analysis of the pathology of photoaging, in which XP where a pigmented macula and a depigmented macula are mixed occurs early in an exposed portion in which a melanocyte is involved, through use of an XP patient-derived melanocyte. In view of the foregoing, the inventors of the present invention have found a method in which a pigment stem cell is produced from a pluripotent stem cell, and a melanocyte is established from the pigment stem cell (Patent Literature 1 and Non Patent Literature 6).

A typical molecular mechanism responsible for epigenetics is, for example, histone modification. A histone acetyltransferase (HAT) and a histone deacetylase (HDAC) are involved in the histone modification. The HAT plays a role in increasing gene expression through the acetylation of the lysine residue of a histone. Meanwhile, the HDAC plays a role in suppressing the gene expression through the deacetylation of the histone. As described above, the HAT and the HDAC each play an important role in the transcriptional control of a gene, and the acetylation modification of the histone is regulated by a competitive action between the HAT and the HDAC.

The HDACs of a human come in 18 kinds, and are classified into four classes ranging from a class I to a class IV each having a feature. The HDACs of the class I and the class II are each a hydrolase having zinc at an enzyme active site thereof, and with regard to intracellular localization, the HDACs have the following features: in the class I, each of the HDACs is localized to a nucleus; and in the class II; each of the HDACs is localized to a nucleus and cytoplasm. HDAC4 belongs to the class II.

### Citation List

### Patent Literature

[PTL 1] JP 6624916 B2

### Non Patent Literature

[NPL 1] Bowden et al. Int. J. Mol. Sci. 2015, 15985-15996
[NPL 2] Herman et al. Environ MolMutagen. 2014 June; 55(5): 375-384
[NPL 3] Fukumoto et al. GenesCells. 2016, Feb; 21(2): 185-99
[NPL 4] Cho-TH et al. PhotodermatolPhotoimmunolPhotomed. 2008 Jun; 24(3): 110-4.
[NPL 5] Emri G et al. J Invest Dermatol. 2000 Sep; 115(3): 435-40
[NPL 6] Hosaka et al. Pigemnt Cell & Melanoma Research, 2019, 32(5): 623-633

### Summary of Invention

### Technical Problem

An object of the present invention is to provide a prophylactic and/or therapeutic agent for a skin disorder associated with a histone deacetylase 4 (HDAC4) abnormality.

### Solution to Problem

The inventors of the present invention have made extensive investigations with a view to achieving the above-mentioned object, and as a result, have found that a histone acetyltransferase inhibitor is effective in preventing and/or treating a skin disorder associated with a histone deacetylase 4 abnormality. Thus, the inventors have completed the present invention.

That is, the present invention includes the following.
1. A prophylactic and/or therapeutic agent for a skin disorder associated with a histone deacetylase 4 abnormality, including a histone acetyltransferase inhibitor as an active ingredient.
2. The prophylactic and/or therapeutic agent for a skin disorder according to the above-mentioned item 1, wherein the skin disorder is a skin disorder associated with a histone deacetylase 4 abnormality in a melanocyte caused by light exposure.
3. The prophylactic and/or therapeutic agent for a skin disorder according to the above-mentioned item 1, wherein the skin disorder is a skin disorder due to photoaging.
4. The prophylactic and/or therapeutic agent for a skin disorder according to the above-mentioned item 1, wherein the skin disorder is a skin disorder due to xeroderma pigmentosum.
5. The prophylactic and/or therapeutic agent for a skin disorder according to the above-mentioned item 1, wherein the prophylactic and/or therapeutic agent for a skin disorder associated with a histone deacetylase 4 abnormality has an alleviating action on an expression abnormality of histone deacetylase 4.
6. The prophylactic and/or therapeutic agent for a skin disorder according to the above-mentioned item 1, wherein the histone acetyltransferase inhibitor is one or a plurality of kinds selected from the group consisting of: curcumin; C-646; A-485; anacardic acid; butyrolactone; CTPB; and garcinol.
7. A method of screening a prophylactic and/or therapeutic agent for a skin disorder, including adding a candidate substance to a culture system of a melanocyte to sort out a substance that alleviates a cell injury of the melanocyte.
8. The screening method according to the above-mentioned item 7, wherein the substance that alleviates the cell injury of the melanocyte is obtained by detecting histone deacetylase 4 and/or a senescence-associated secretory phenotype factor.
9. The screening method according to the above-mentioned item 7, wherein the substance that alleviates the cell injury of the melanocyte is obtained by evaluating a survival rate of the melanocyte.
10. The screening method according to the above-mentioned item 7, wherein the substance that alleviates the cell injury of the melanocyte is obtained by evaluating a cell migration ability of the melanocyte.
11. The screening method according to the above-mentioned item 7, wherein the melanocyte is a pluripotent stem cell-derived melanocyte.
12. The screening method according to the above-mentioned item 11, wherein the pluripotent stem cell-derived melanocyte is a pluripotent stem cell-derived melanocyte produced from a pluripotent stem cell derived from a xeroderma pigmentosum patient.
13. The screening method according to the above-mentioned item 7, wherein the cell injury of the melanocyte is a cell injury due to light exposure.
14. The screening method according to the above-mentioned item 13, wherein the cell injury due to the light exposure is a skin disorder associated with a histone deacetylase 4 abnormality.
15. A method of testing a skin disorder, including a step of detecting an abnormality of histone deacetylase 4 and/or a senescence-associated secretory phenotype factor from skin collected from a subject.
16. The method of testing a skin disorder according to the above-mentioned item 15, wherein the senescence-associated secretory phenotype factor is one or a plurality of kinds selected from MMP-1, MMP-3, TNFα, and IL-6.
17. The method of testing a skin disorder according to the above-mentioned item 15, wherein the detecting the abnormality of histone deacetylase 4 and/or the senescence-associated secretory phenotype factor is performed by using the following (1) or (2): (1) a nucleic acid probe or a nucleic acid primer capable of specifically detecting a transcription product of a histone deacetylase 4 gene and/or a senescence-associated secretory phenotype factor gene; and (2) an antibody that specifically recognizes histone deacetylase 4 and/or the senescence-associated secretory phenotype factor.
18. A kit for testing a skin disorder, including a substance capable of detecting an abnormality of histone deacetylase 4 and/or a senescence-associated secretory phenotype factor.

### Advantageous Effects of Invention

The use of the prophylactic and/or therapeutic agent for a skin disorder associated with a histone deacetylase 4 abnormality of the present invention, the agent including the histone acetyltransferase inhibitor as an active ingredient, can prevent and/or treat the skin disorder associated with the histone deacetylase 4 abnormality.

### Brief Description of Drawings

FIG. 1 is a set of photographs showing melanocytes (HC-iMCs) differentiated from normal fibroblast-derived iPS cells derived from human skin and melanocytes (XP-A-iMCs) differentiated from fibroblast-derived iPS cells derived from an XP-A patient (Reference Example 1).
FIG. 2 is a set of photographs showing the expression of SOX10, MITF, and TYR serving as MC markers in the respective melanocytes (Reference Example 1).
FIG. 3A is a set of graphs showing mutations by genomic sequencing in the XP-A-iMCs and the HC-iMCs. FIG. 3B shows that the XP-A-iMCs do not express any XPA protein (Example 1).
FIGS. 4 show that the XP-A-iMCs have the properties of XP-A. FIG. 4A is a set of graphs showing results in a flow cytometry-based NER assay. FIG. 4B is a set of graphs showing results in an MTT assay (Example 2).
FIG. 5A is a diagram for illustrating a protocol for the irradiation of the XP-A-iMCs and the HC-iMCs with UV light. FIG. 5B is a graph showing a difference between the XP-A-iMCs and the HC-iMCs, which are affected by the UV light, in a gene. FIG. 5C is a set of graphs showing probes whose gene expression levels increase or reduce after the irradiation of the XP-A-iMCs and the HC-iMCs with high-dose UV-B (Example 3).
FIG. 6 is a set of graphs showing the results of GO analysis for elucidating a molecular mechanism related to the cell response of the XP-A-iMCs to the UV light (Example 3).
FIG. 7 is a graph showing a change in HDAC4 expression of the XP-A-iMCs after the UV irradiation (Example 4).
FIGS. 8 are graphs showing the results of the HDAC4 expression of the XP-A-iMCs after the UV irradiation (Example 4).
FIG. 9 is a set of photographs showing the results of a scratch assay in the XP-A-iMCs after the UV irradiation with a phase-contrast microscope (Example 5).
FIG. 10 is a graph showing the results of the examination of cell migration abilities by the scratch assay in the XP-A-iMCs after the UV irradiation (Example 5).
FIG. 11 is a diagram for illustrating a method of observing cell migration in the XP-A-iMCs after the UV irradiation by digital holographic microscopy (Example 5).
FIG. 12 is a graph showing the distance by which cells have migrated in the XP-A-iMCs after the UV irradiation, the distance being measured by the digital holographic microscopy (Example 5).
FIG. 13 is a graph showing the speed of the cell migration in the XP-A-iMCs after the UV irradiation measured by the digital holographic microscopy (Example 5).
FIG. 14 is a set of photographs showing the results of the examination of the cellular senescence of the XP-A-iMCs after the UV irradiation (Example 6).
FIG. 15 is a set of graphs showing the results of the expression of a SASP factor group in the XP-A-iMCs after the UV irradiation by a PCR method (Example 6).
FIG. 16 is a set of graphs showing the results of the expression of the SASP factor group in the XP-A-iMCs after the UV irradiation by a western blotting method (Example 6).
FIG. 17 is a graph showing the results of the HDAC4 expression of NHEMs after their UV irradiation (Example 7).
FIG. 18 is a set of graphs showing the effects of curcumin in the XP-A-iMCs after the UV irradiation (Example 8).
FIG. 19 is a graph showing the effects of C-646 in the XP-A-iMCs after the UV irradiation (Example 9).

### Description of Embodiments

The present invention relates to a prophylactic and/or therapeutic agent for a skin disorder associated with a histone deacetylase 4 (HDAC4) abnormality, the agent including a histone acetyltransferase (HAT) inhibitor as an active ingredient.

The inventors of the present invention have searched for a reaction to light exposure in a melanocyte, have comprehensively analyzed molecules that may be therapeutic target seeds related to a skin disorder, and have identified HDAC4 as a gene that epigenetically controls the melanocyte. Further, the inventors have revealed that cellular senescence occurs in the melanocyte after the light exposure through use of an immunofluorescent antibody method. Further, the inventors have conceived that the melanocyte that has caused the cellular senescence affects a senescence-associated secretory phenotype (SASP) factor group, and have actually recognized an increase in expression of each of many SASP factor groups. That is, the following is conceivable: the expression of HDAC4 in skin is reduced by the light exposure to relatively cause the acetylation of a histone; and hence, the expression of a SASP-related gene increases along with the foregoing to cause various skin disorders.

In view of the foregoing, the inventors of the present invention have found for the first time a possibility that the alleviation of the expression abnormality of HDAC4 is applicable to the prevention and/or treatment of a skin disorder. In this description, a senescence-associated secretory phenotype (SASP) factor is, for example, MMP-1, MMP-3, TNFα, or IL-6.

The term "HDAC4 abnormality" as used herein refers to the quantitative abnormality of histone deacetylase 4 (HDAC4), and particularly refers to a state in which the expression of HDAC4 reduces after its light exposure as compared to that before the light exposure. The term "expression" may refer to expression at a gene level, or may refer to expression at a protein level. The prophylactic and/or therapeutic agent of the present invention is characterized by having an alleviating action on the expression abnormality of HDAC4.

The term "skin disorder" as used herein is particularly applied to a skin disorder associated with a HDAC4 abnormality, and examples thereof include: a skin disorder associated with a HDAC4 abnormality in a melanocyte caused by light exposure; a skin disorder due to photoaging; and a skin disorder due to xeroderma pigmentosum. The term "photoaging" as used herein means changes in appearance and function of skin observed as a result of the repetition of light exposure, and includes a wide variety of skin disorders that may be caused by the light exposure. Specific skin disorders are not limited to skin disorders that may occur in the skin of an XP patient, and include a skin disorder that may be caused in a person who is not an XP patient by light exposure. Examples of the skin disorder include, but not limited to, a pigment lesion in which a pigmented macula and a depigmented macula are mixed, the drying and atrophy of skin, a reduction in elasticity of the skin, the formation of a wrinkle or a sag, skin cancer that occurs from a young age, the formation of a freckle, pigmentation, the blackening of the skin and an increase in yellow tint thereof, a reduction in skin barrier function, and a reduction in horny layer function.

As described in the "Background Art" section, the term "xeroderma pigmentosum (XP)" as used herein refers to a hereditary photosensitive disease that is inherited in an autosomal recessive manner. The term "XP" as used herein is used in a concept including all of the following eight disease types: a group A to a group G (genetically complementary groups) that are genetically different from each other, and a variant (V) type. The term is particularly suitably applied to the group A (XP-A), which is such a type that both of a skin symptom and a neurological symptom are most severe. Research in a melanocyte is important in the advance of the pathological elucidation of XP particularly because dyschromatosis is inevitable.

The term "light exposure" as used herein is not limited to typical light exposure in daily life or light exposure exceeding the typical light exposure (e.g., sunburn), and includes slight light exposure that may cause a skin disorder in an XP patient. The term "light" as used herein is not particularly limited as long as the "light" includes UV light, and examples thereof include sunlight, UV light (e.g., UV-B, UV-C, or UV-A), and illumination light. The term particularly refers to sunlight or UV light. Although the wavelength of the light is not particularly limited, the wavelength is, for example, from 0 nm to 3,000 nm, preferably from 10 nm to 400 nm, more preferably from 200 nm to 380 nm, particularly preferably from 280 nm to 315 nm. Although the dose thereof is not particularly limited, the dose is, for example, from 10 J/m² to 500 J/m², preferably from 30 J/m² to 200 J/m², more preferably 150 J/m².

The term "HAT inhibitor" as used herein literally refers to a substance that inhibits or suppresses the enzyme activity of a histone acetyltransferase (HAT). The inhibition or suppression of the enzyme activity of the HAT relatively provides a balance between HDAC4 and the HAT to alleviate the abnormality of HDAC4. The HAT inhibitor is, for example, one or a plurality of kinds selected from the group consisting of: curcumin; C-646; A-485; anacardic acid; butyrolactone; CTPB; and garcinol. The prophylactic and/or therapeutic agent of the present invention includes the HAT inhibitor as an active ingredient. In addition to compounds, such as curcumin, C-646, A-485, anacardic acid, butyrolactone, CTPB, and garcinol, the HAT inhibitor to be incorporated as an active ingredient in this description may be a derivative of each of the compounds, or a pharmaceutically acceptable salt of the compound or of the derivative of the compound, or may be a hydrate thereof. Of those, curcumin, C-646, or A-485 is particularly suitable. Curcumin (IUPAC name: (1E,6E)-1,7-bis(4-hydroxy-3-methoxyphenyl)-1,6-heptadiene-3,5-dione) is a fat-soluble polyphenol, which is mainly incorporated at a high concentration into turmeric, and is also incorporated into, for example, cardamom, clove, cumin, pepper, coriander, paprika, or mace. Known examples of the potential of the medical use of curcumin include an antitumor action, an antioxidant action, an anti-amyloid action, and an anti-inflammatory action, and hence curcumin has been vigorously studied in recent years.

The prophylactic and/or therapeutic agent of the present invention is used as a pharmaceutical composition. The pharmaceutical composition means a composition suitable for administration in medical applications, and may contain one or a plurality of kinds of pharmaceutically acceptable carriers and/or pharmaceutically acceptable excipients in addition to the HAT inhibitor serving as an active ingredient. The pharmaceutical composition serving as the prophylactic and/or therapeutic agent of the present invention may be prepared by a method typically used in the art through use of, for example, a carrier or excipient for a drug typically used therein. The administration may be in the form of any one of: oral administration by, for example, a tablet, a pill, a capsule, a granule, a powder, or a liquid formulation; and parenteral administration by, for example, an intraarticular, intravenous, or intramuscular injection, a suppository, an eye drop, an eye ointment, a transdermal liquid formulation, an ointment, a transdermal patch, a transmucosal liquid formulation, a transmucosal patch, or an inhalant. A tablet, a powder, a granule, a sustained-release agent, or the like is used as a solid composition for oral administration according to the present invention. In such solid composition, one or two or more kinds of active ingredients are mixed with at least one kind of pharmaceutically acceptable carrier and/or pharmaceutically acceptable excipient.

Examples of the pharmaceutically acceptable carrier of this description include various organic or inorganic carrier substances commonly used as formulation materials. Examples thereof include: an excipient, a lubricant, a binder, and a disintegrant in a solid formulation; and a solvent, a solubilizing agent, a suspending agent, an isotonizing agent, and a buffering agent in a liquid formulation. Further, an appropriate amount of a typical additive, such as an antiseptic, an antioxidant, a colorant, a sweetener, an adsorbent, or an wetting agent, may be appropriately used as required.

Examples of the pharmaceutically acceptable excipient of this description include an isotonizing agent, a bulking agent, an antiseptic bactericide, a binder, an oxidation inhibitor, a solubilizer, a solubilizing agent, a suspending agent, a filler, a pH regulator, a stabilizer, an absorption promoter, a release rate-controlling agent, a colorant, a plasticizer, and a pressure-sensitive adhesive. Specific examples thereof include lactose, white sugar, D-mannitol, starch, corn starch, crystalline cellulose, and light anhydrous silicic acid.

Examples of the lubricant include magnesium stearate, calcium stearate, talc, and colloidal silica. Examples of the binder include crystalline cellulose, white sugar, D-mannitol, dextrin, hydroxypropylcellulose, hydroxypropylmethylcellulose, polyvinylpyrrolidone, starch, sucrose, gelatin, methylcellulose, and carboxymethylcellulose sodium.

Examples of the disintegrant include starch, carboxymethylcellulose, carboxymethylcellulose calcium, croscarmellose sodium, carboxymethyl starch sodium, and L-hydroxypropylcellulose.

Examples of the solvent include water for injection, an alcohol, propylene glycol, a macrogol, sesame oil, corn oil, and olive oil.

Examples of the solubilizing agent include polyethylene glycol, propylene glycol, D-mannitol, benzyl benzoate, ethanol, trisaminomethane, cholesterol, triethanolamine, sodium carbonate, and sodium citrate.

Examples of the suspending agent include: surfactants, such as stearyl triethanolamine, sodium lauryl sulfate, laurylaminopropionic acid, lecithin, benzethonium chloride, and glycerin monostearate; and hydrophilic polymers, such as polyvinyl alcohol, polyvinylpyrrolidone, carboxymethylcellulose sodium, methylcellulose, hydroxymethylcellulose, hydroxyethylcellulose, and hydroxypropylcellulose.

Examples of the isotonizing agent include glucose, D-sorbitol, sodium chloride, glycerin, and D-mannitol.

Examples of the buffering agent include buffer solutions of a phosphate, an acetate, a carbonate, and a citrate.

Examples of the antiseptic include paraoxybenzoic acid esters, chlorobutanol, benzyl alcohol, phenethyl alcohol, dehydroacetic acid, and sorbic acid.

Examples of the antioxidant include a sulfite, ascorbic acid, and α-tocopherol.

The administration amount of the HAT inhibitor serving as an active ingredient varies depending on, for example, its administration target and administration route, and the symptom, body weight, and age of the target, and may be appropriately selected. The HAT inhibitor serving as an active ingredient may be appropriately used and administered in combination with any other drug.

The present invention covers a method of screening a prophylactic and/or therapeutic agent for a skin disorder. The screening method may be characterized by including adding a candidate substance to the culture system of a melanocyte to sort out a substance that alleviates the cell injury of the melanocyte.

The term "melanocyte" (also referred to as "pigment cell" or "melanin cell") means the following cell: the cell has tyrosinase activity and produces a melanin; the cell has an intracellular organelle (melanosome) for melanin synthesis; and the cell is a dendritic cell in morphology and the shapes of its dendrites change with an environment.

The melanocyte may be produced from, for example, a pluripotent stem cell. Examples of the pluripotent stem cell include an induced pluripotent stem cell (iPS cell), an embryonic stem cell (ES cell), an embryonic carcinoma cell (EC cell), and an embryonic germ stem cell (EG cell). Of those, an iPS cell is preferred. A method known per se or any one of various methods to be developed in the future is applicable as a method of inducing differentiation from the pluripotent stem cell into the melanocyte. Specifically, a method described in Examples or a method described in JP 6624916 B2 is applicable. An iPS cell that has already been established may be used as the pluripotent stem cell, or the cell may be produced by a method known per se or any one of various methods to be developed in the future. Specific examples thereof include a method described in Examples, and methods described in Cell, 131(5): 861-872 (2007), Cell, 126(4): 663-676 (2006), and Non Patent Literature 6. The pluripotent stem cell may be produced from, for example, an XP patient-derived cell. A method known per se or any one of various methods to be developed in the future is applicable as a method of producing an XP patient-derived pluripotent stem cell. Specifically, a method described in Examples or a method described in JP 2021-17405 A is applicable. An environment in which the melanocyte is cultured only needs to be an environment known per se, and is hence not particularly limited. However, the cell may be generally cultured under the conditions of 37±1°C and 5±1% CO₂.

The term "cell injury of the melanocyte" as used herein suitably refers to, for example, a cell injury due to light exposure. Although the melanocyte is responsible for the defense of skin from light through melanin production, the melanocyte itself causes DNA damage owing to UV light, and hence the expression abnormality of the gene HDAC4 that controls the acetylation of DNA occurs. The term "cell injury due to light exposure" suitably refers to a skin disorder associated with a HDAC4 abnormality. In the screening method of the present invention, the substance that alleviates the cell injury of the melanocyte may be sorted out by a method known per se or any one of various methods to be developed in the future. The substance that alleviates the cell injury of the melanocyte may be sorted out by a method, such as the detection of HDAC4 and/or the SASP factor, the evaluation of the survival rate of the melanocyte, or the evaluation of the cell migration ability of the melanocyte. In such sorting method, the substance that alleviates the cell injury of the melanocyte may be sorted out by comparing the substance to any other substance on the basis of the presence or absence of light exposure. The candidate substance to be subjected to the screening method of the present invention may be a high-molecular weight compound, or may be a low-molecular weight compound. Examples of the high-molecular weight compound include, but not particularly limited to, a protein and a nucleic acid substance, and the compound is specifically, for example, an antibody, an antibody fragment, or a peptide, such as siRNA or shRNA. Examples of the low-molecular weight compound are not particularly limited. A substance containing the low-molecular weight compound and the high-molecular weight compound is also permitted.

The present invention covers a method of testing a skin disorder including a step of detecting the abnormality of HDAC4 and/or a SASP factor from skin collected from a subject.

The step of detecting the abnormality of HDAC4 and/or the SASP factor is not particularly limited as long as the step is a step of detecting the abnormality of HDAC4 and/or the SASP factor by a method by which the abnormality can be detected. Examples thereof include: a step of preparing an RNA (e.g., total RNA or mRNA) fraction from the skin of the subject, followed by the detection of the transcription product of a HDAC4 gene and/or a SASP factor gene in the fraction; a step of detecting the abnormality by an immunological approach including using an antibody that specifically recognizes HDAC4 and/or the SASP factor; and a step of detecting the abnormality by mass spectrometry. Specifically, in the test method of the present invention, the abnormality of HDAC4 and/or the SASP factor may be detected by using the following (1) or (2):
(1) a nucleic acid probe or a nucleic acid primer capable of specifically detecting the transcription product of the HDAC4 gene and/or the SASP factor gene; and
(2) an antibody capable of specifically recognizing HDAC4 and/or the SASP factor.

Although the RNA fraction may be prepared by using a known approach, such as a guanidine-CsCl ultracentrifugation method or an AGPC method, high-purity total RNA may be quickly and simply prepared from a trace amount of a specimen by using a commercially available kit for RNA extraction (e.g., RNeasy Mini Kit; manufactured by QIAGEN N.V.). A method of detecting the transcription product of the HDAC4 gene and/or the SASP factor gene in the RNA fraction is, for example, a method including using a PCR (e.g., a RT-PCR, a competitive PCR, or a real-time PCR) or hybridization (e.g., a northern blot, a dot blot, or DNA chip analysis). Examples of the immunological approach include a western blotting method, an ELISA, a FIA, and a RIA. The term "antibody" as used herein includes, but not limited to, a natural antibody, such as a polyclonal antibody or a monoclonal antibody (mAb), a chimeric antibody, a humanized antibody, or a single-chain antibody that may be produced by using a gene recombination technology, and binding fragments thereof. The antibody is preferably a polyclonal antibody, a monoclonal antibody, or a binding fragment thereof. The term "binding fragment" means a partial region of each of the above-mentioned antibodies having specific binding activity, and specific examples thereof include F(ab')₂, Fab', Fab, Fv, sFv, dsFv, and sdAb (Exp. Opin. Ther. Patents, Vol. 6, No. 5, p. 441-456, 1996). The class of the antibody is not particularly limited, and comprehends an antibody having any isotype, such as IgG, IgM, IgA, IgD, or IgE. Of those, IgG or IgM is preferred, and in consideration of, for example, ease of purification, IgG is more preferred. In addition, in the present invention, a commercially available anti-HDAC4 antibody or anti-SASP factor antibody, or a commercially available anti-HDAC4 antibody- or anti-SASP factor antibody-containing kit is preferably used as an antibody that specifically recognizes HDAC4 and/or the SASP factor.

In the test method of the present invention, when HDAC4 is detected at a lower value in the subject than that in a control or when the SASP factor is detected at a higher value therein than that in the control, it can be judged that there is a high possibility of such skin disorder as described above. Accordingly, the test method of the present invention may include (3) a step of judging that a skin disorder occurs or there is a high possibility thereof when HDAC4 is detected at a lower value in the subject than that in the control or the SASP factor is detected at a higher value therein than that in the control in addition to the above-mentioned steps (1) and (2).

In the test method of the present invention, the term "subject" includes, for example, such a person that whether or not he or she suffers from XP is unclear and a person in whom XP is suspected in addition to, for example, a person in whom whether or not a skin disorder occurs is unclear and a person in whom a skin disorder is suspected.

The present invention further covers a kit for testing a skin disorder including a substance capable of detecting the abnormality of HDAC4 and/or a SASP factor. Examples of the substance capable of detecting the abnormality of HDAC4 and/or the SASP factor include: a nucleic acid probe or a nucleic acid primer capable of specifically detecting the transcription product of a HDAC4 gene and/or a SASP factor gene; and an antibody capable of specifically recognizing HDAC4 and/or the SASP factor. In this description, the "primer, probe, or the like" may be modified as long as its function is not remarkably impaired. Examples of the modification include a labeling substance, a fluorescent dye, an enzyme, a protein, a radioisotope, a chemiluminescent substance, and biotin. The primer, the probe, or the like may be used by being immobilized to any solid phase. In this description, the "solid phase" is not particularly limited as long as the phase can immobilize a polynucleotide, and the phase is, for example, a glass plate, a nylon membrane, microbeads, a silicon chip, a capillary, or any other substrate. The kit of the present invention may further include, for example, a reaction buffer solution, dNTPs, a heat-resistant DNA polymerase, a competitor nucleic acid, a fluorescent reagent, a competitor antibody, a labeled secondary antibody, or a blocking liquid.

### Examples

To help the understanding of the present invention, the present invention is specifically described below by way of Reference Example and Examples, but it goes without saying that the present invention is not limited thereto. In "Reference Example," the contents of an experiment leading to the completion of the present invention are described.

### (Reference Example 1) Production of iPS-derived Melanocytes

In this Reference Example, an XP disease-specific pigment stem cell was produced, and was then differentiated into a melanocyte (MC) to produce an iPS-derived melanocyte.

### (Production of XP Disease-specific Pigment Stem Cells)

Human skin-derived normal fibroblasts (TIG-120 cells; JCRB0542) and XP-A patient-derived fibroblasts (XP30S) were each used in the establishment of a human iPS cell strain. The XP30S cells (JCRB0303) are cells known to have the Japanese founder mutation of XP-A (c.390-1G>C:g.15148G>C). Those cell strains were obtained from JCRB Bioresource Bank (Japan), and were cultured in a medium obtained by adding 10% fetal bovine serum and 1% penicillin/streptomycin (Life Technologies Corporation) to Dulbecco's modified Eagle's medium (DMEM) (Nacalai Tesque, Inc.). The humane iPS cell strain was established from each of those two fibroblast strains by using CytoTune-iPS 2.0 Sendai Reprogramming Kit (ID Pharma Co., Ltd.) (Non Patent Literature 6). After that, human iPS cells were seeded onto a non-adherent culture dish (50 to 200 cells/ml), and were cultured in a medium for primate ES/iPS cells for 2 weeks to form an embryoid body. After that, the embryoid body was seeded onto a fibronectin-coated adherent culture dish, and was cultured in a melanocyte maintenance medium (medium obtained by adding Human Melanocyte Growth Supplement (HMGS) (Invitrogen) to Medium 254 (Invitrogen)) having added thereto 3 µM CHIR99021 (Stemgent), 50 ng/ml SCF (R&D Systems), 100 nM ET-3 (American Peptide Company, Inc.), 500 µM dbcAMP (Sigma-Aldrich Co. LLC), 50 nM 12-tetradecanoylphorbol 13-acetate (TPA) (Sigma-Aldrich Co. LLC), 4 ng/ml bFGF (Wako Pure Chemical Industries, Ltd.), 100 µM L-ascorbic acid (Sigma-Aldrich Co. LLC), 0.05 µM dexamethasone (Sigma-Aldrich Co. LLC), 1 mg/ml linoleic acid-bovine serum (Sigma-Aldrich Co. LLC), and 1X insulin-transferrin-sodium selenite (Sigma-Aldrich Co. LLC) so that the differentiation of the embryoid body into melanocytes was induced. During the culture, the medium was replaced with a new one once per 2 to 3 days. 14 Days after the differentiation induction, cells containing spindle-shaped and blackish brown granules were observed with a microscope. Accordingly, the differentiation induction was stopped, and the medium was switched to a melanocyte maintenance medium free of any differentiation-inducing factor, followed by the culture of the cells. Seven days after the stop of the differentiation induction, pigment stem cells were observed in the medium.

### (Induction of Differentiation into XP Disease-specific Melanocytes)

Three micromolar CHIR99021 was added to the above-mentioned pigment stem cells every 2 days. About one week after the start of the addition, the cells were differentiated into MCs. Cells obtained by differentiating the XP-A patient-derived fibroblast-derived iPS cells into the melanocytes are hereinafter referred to as "XP-A-iMCs", and cells obtained by differentiating the human skin-derived normal fibroblast-derived iPS cells into the melanocytes are hereinafter referred to as "HC-iMCs".

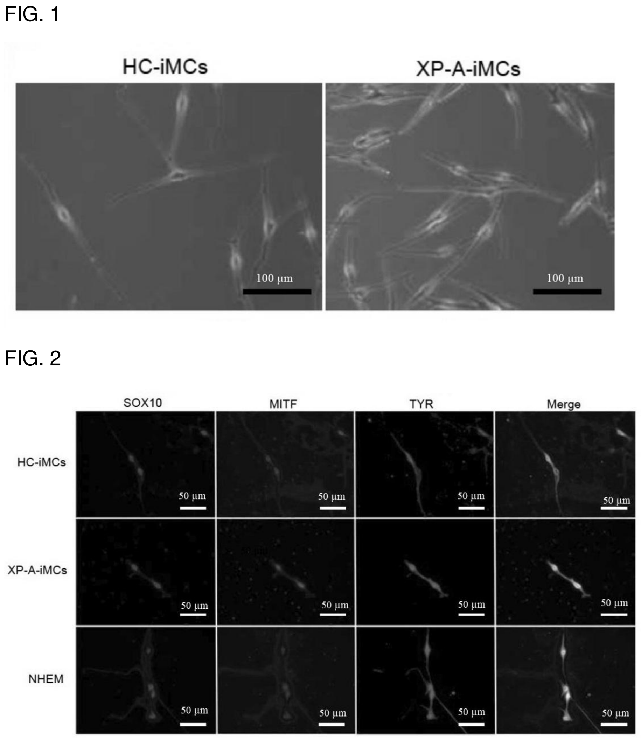

The XP-A-iMCs and the HC-iMCs were observed with a phase-contrast microscope. As a result, the XP-A-iMCs and the HC-iMCs had elongated spindle shapes having dendrites as in normal human epidermal melanocytes (NHEMs) (FIG. 1). In addition to the visual observation of the cell shapes, to promise further precision, the observation of a pellet by centrifugal separation and immunohistochemical staining were performed. The observation of the pellet by the centrifugal separation showed that the cells were differentiated into MCs because the cells had black color tones when visually observed. In the immunohistochemical staining, it was recognized from the expression of SOX10, MITF, or TYR serving as a MC marker that the cells had the properties of MCs (FIG. 2).

### (Example 1) Recognition of Mutation by Genomic Sequencing

In this Example, whether or not the XP-A-iMCs correctly had a Japanese founder mutation (c.390-1G>C:g.15148G>C) was recognized.

Genomic DNA was separated from the XP-A-iMCs with Gentra Puregene Cell Kit (QIAGEN N.V., Hilden). To recognize the presence or absence of the Japanese XP-A founder mutation g.15148G>C of an XP-A gene (c.390-1 G>C), a region including the mutation was amplified with each of the following PCR primers. 5'-GCTGTGTGCCTAAGTT-3' (forward) (SEQ ID NO: 1) 5'-TGCAAAACTAGGAAAAGTTT-3' (reverse) (SEQ ID NO: 2) Ex-Taq (Takara Bio Inc.)

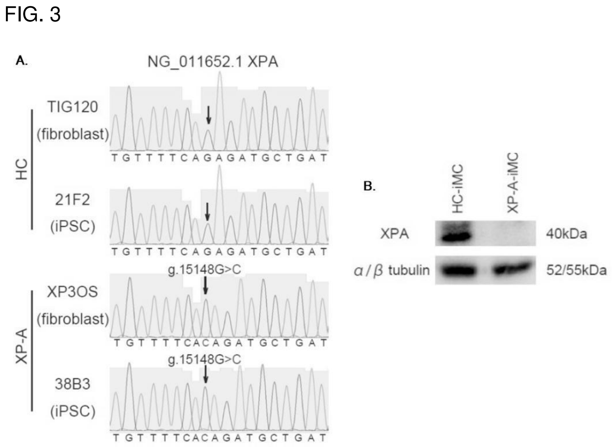

A PCR product was decomposed with agarose gel, and was purified with QIA quick Gel Extraction Kit (QIAGEN N.V.), followed by the determination of its sequence through use of Applied Biosystems 3500 Genetic Analyzer (Applied Biosystems) and the same forward primer (FIG. 3A). Further, it was recognized by a western blotting method that the XP-A-iMCs did not express any XPA protein (FIG. 3B).

### (Example 2) Recognition of Fact that XP-A-iMCs have Properties of XP-A

In this Example, the fact that the XP-A-iMCs had the following property as a property of XP was examined by a flow cytometry-based NER assay (Nakano et al, 2018, J Invest Dermatol, 138: 467-47) and an MTT (proliferation/survival) assay: the XP-A-iMCs were brittle to UV light because DNA repair was hindered. The flow cytometry-based NER assay is a DNA repair test used in clinical diagnosis, and is a method based on analysis by flow cytometry through use of a monoclonal antibody against a (6-4) PP after its irradiation with UV-C.

### (Flow Cytometry-based NER Assay)

The DNA lesion repair of each of the XP-A-iMCs and the HC-iMCs was evaluated by the flow cytometry-based NER assay. Specifically, the XP-A-iMCs and the HC-iMCs were seeded into 10-centimeter dishes, and were irradiated with UV-C at a dose of 30 J/m² the next day. Immediately after the irradiation or 6 hours after the irradiation, the XP-A-iMCs and the HC-iMCs were collected. After that, the XP-A-iMCs and the HC-iMCs were stained with a mouse primary monoclonal antibody (1:1,000, Cosmo Bio Co., Ltd.) against the (6-4) PP at 22°C for 1.5 hours, and were resuspended in PBS-TB containing an Alexa Fluor 488 antibody (1:200, Thermo Fisher Scientific, Inc.) at 22°C for 1 hour. Further, the XP-A-iMCs and the HC-iMCs were stained by being resuspended in PBS containing 5 µg/ml propidium iodide (Thermo Fisher Scientific, Inc.). After the staining, the XP-A-iMCs and the HC-iMCs were analyzed with a FACS Verse flow cytometer (BD Biosciences). All data was analyzed with Flow Jo software (BD Life Sciences). The removal ratio of a DNA lesion was calculated on the basis of the average fluorescence intensity of each of the XP-A-iMCs and the HC-iMCs. The fluorescence intensities of the XP-A-iMCs and the HC-iMCs that were not irradiated with UV light were used as backgrounds, and the fluorescence intensity of each of the cells immediately after their UV irradiation was defined as 100%. A reduction in fluorescence intensity thereof was measured 6 hours after the irradiation.

### (MTT Assay)

A cell survival rate was identified by the MTT assay. Specifically, the XP-A-iMCs, the HC-iMCs, and the NHEMs 24 hours after their irradiation with UV-B were evaluated for their survival rates by using Cell Proliferation Kit I (MTT assay) (F. Hoffmann-La Roche, Ltd.) in accordance with the protocol of the manufacturer. The medium of the melanocytes was replaced with a phenol red-free medium (Lifeline Cell Technology) immediately before a reaction with an MTT reagent. Then, the final product was transferred to a 96-well plate, and its cell survival rates were measured with an Emax precision microplate reader (Molecular Devices) at absorbances of 595 nm and 650 nm. Each of the cell survival rates was represented as a value relative to the number of the melanocytes that were not irradiated with UV light.

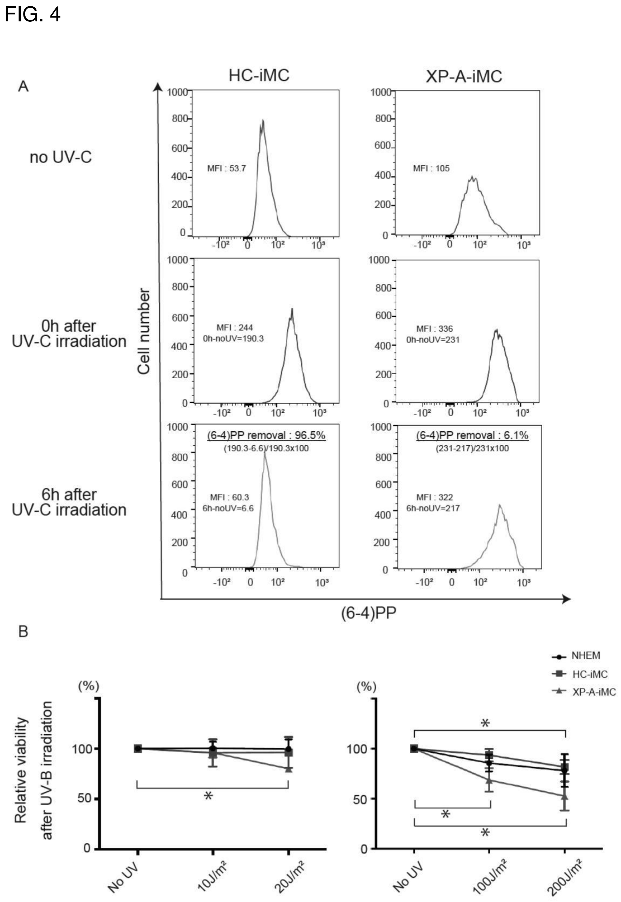

The results in the flow cytometry-based NER assay are shown in FIG. 4A. FIG. 4A shows typical histograms showing the relative signal intensities of the (6-4) PP without irradiation, immediately after the UV-C irradiation, and 6 hours after the UV-C irradiation. It was shown that in the HC-iMCs and the XP-A-iMCs, the (6-4) PP was repaired by 96.5% and 6.1%, respectively within 6 hours after the UV-C irradiation. Similar results were obtained from 3 independent experiments.

The results in the MTT assay are shown in FIG. 4B. The cell survival rates of the XP-A-iMCs and the HC-iMCs 24 hours after the irradiation were evaluated at each of low doses (10 J/m² and 20 J/m²) and high doses (100 J/m² and 200 J/m²) (FIG. 4B). As reference data, the survival rate of commercially available NHEMs was also evaluated (Gaddameedhi S et al 2010). The survival rate of the HC-iMCs was substantially the same as the survival rate of the NHEMs, and significantly reduced at 200 J/m² as compared to that at the time of a baseline, but did not reduce at 100 J/m². Meanwhile, the survival rate of the XP-A-iMCs significantly reduced when the HC-iMCs were irradiated with UV-B at a dose of 20 J/m² or more.

### (Example 3) Research on Pathological Elucidation of XP with XP-A-iMCs

In this Example, the UV response of the XP-A-iMCs was analyzed, reactions to UV stimuli in melanocytes were comprehensively searched for, and the identification of molecules (drug target molecules) capable of serving as therapeutic target seeds by being directly involved in the pathological formation of XP was examined.

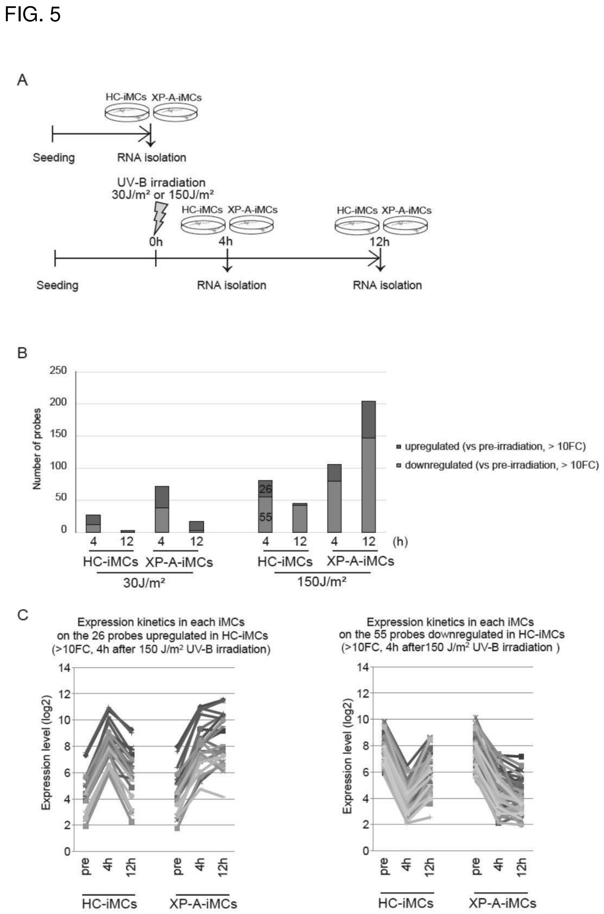

The XP-A-iMCs and the HC-iMCs were seeded into 10-centimeter dishes, and were each irradiated with UV-B at a low dose (30 J/m²) or a high dose (150 J/m²). After that, the XP-A-iMCs and the HC-iMCs were incubated at 37°C for 4 hours or 12 hours (FIG. 5A), and were then comprehensively subjected to transcriptional profile analysis. The gene transcriptional profiling was performed by using GeneChip^{™} Human Genome U133 Plus 2.0 Array (Affymetrix, Inc.) in accordance with the protocol of the manufacturer. Data was analyzed by using a robust multichip average (RMA) method and GeneSpring 13.1.1 software (Agilent Technologies, Inc.). A difference between the XP-A-iMCs and the HC-iMCs, which were affected by the UV-B, in a gene was elucidated.

The number of probes in each of which the gene expression level of each of the XP-A-iMCs and the HC-iMCs significantly changed (by a factor of 10 or more) is shown in FIG. 5B. The examination of the number of the probes in each of which the expression level significantly changed (by a factor of 10 or more) found that when the HC-iMCs were irradiated with low-dose UV-B, the expression levels of 27 probes changed (upregulated: 15, downregulated: 12). However, it was revealed that although the expression level of each gene changed by a factor of 10 or more 4 hours after the irradiation as compared to that before the irradiation, the levels of substantially all the genes returned to those before the irradiation 12 hours after the irradiation. Meanwhile, when the XP-A-iMCs were irradiated with the low-dose UV-B, the expression levels of 72 probes significantly changed (upregulated: 34, downregulated: 38) 4 hours after the irradiation. The number was considerably larger than that observed in the HC-iMCs.

Next, the HC-iMCs were irradiated with high-dose UV-B (150 J/m²), and 4 hours after that, 26 or 55 probes in which their expression levels increased or reduced by a factor of 10 or more were picked up. The gene expression levels of those probes in the HC-iMCs and the XP-A-iMCs 4 or 12 hours after the irradiation are shown in FIG. 5C. In the HC-iMCs, all the expression levels returned to the original levels 12 hours after the irradiation. Meanwhile, it was found that in the XP-A-iMCs, the expression levels of substantially all the probes did not return to the basal levels even 12 hours thereafter, and remained upregulated or downregulated (FIG. 5C). It was revealed that the XP-A-iMCs had the following feature (FIG. 5C): their gene expression level did not return to the level before the irradiation even after the lapse of 12 hours from the irradiation.

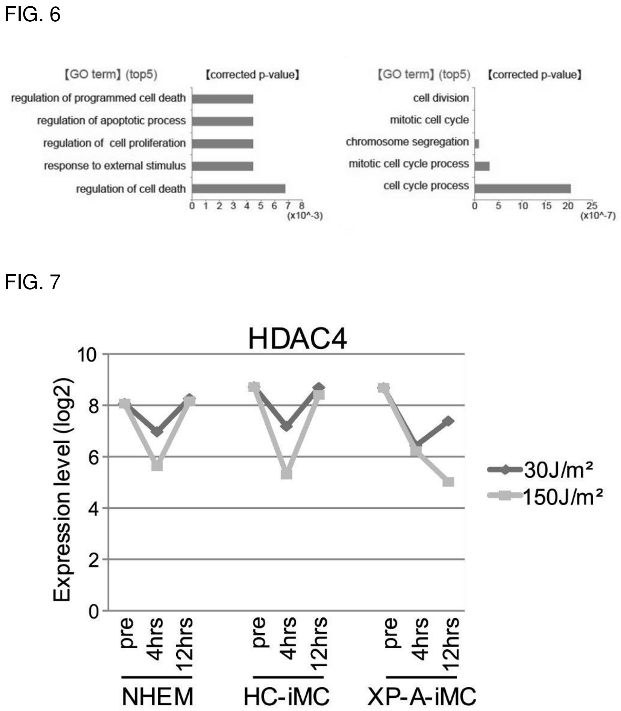

To elucidate a molecular mechanism related to the cell response of the XP-A-iMCs to UV-B in more detail, GO analysis (p<0.05) was performed. The results of genes (n=49) whose expression specifically increased in the XP-A-iMCs 12 hours after their irradiation with the high-dose UV-B showed that the regulation of cell proliferation and cell death belonged to the main GO term category of the XP-A-iMCs, and the category included the regulation of programmed cell death (p=0.004), the regulation of an apoptotic process (p=0.004), the regulation of the cell proliferation (p=0.004), a response to a stimulus (p=0.004), and the regulation of the cell death (p=0.007). Meanwhile, the main GO term category of the XP-A-iMCs suggested from 132 genes whose expression specifically reduced included cell division (p=3.15×10⁻¹⁰), a mitotic cell cycle (p=3.88×10⁻⁹), chromosome segregation (p=8.64×10⁻⁸), a mitotic cell cycle process (p=3.12×10⁻⁷), and a cell cycle process (p=2.04×10⁻⁶) (FIG. 6) .

### (Example 4) Identification of Histone Deacetylase 4 (HDAC4)

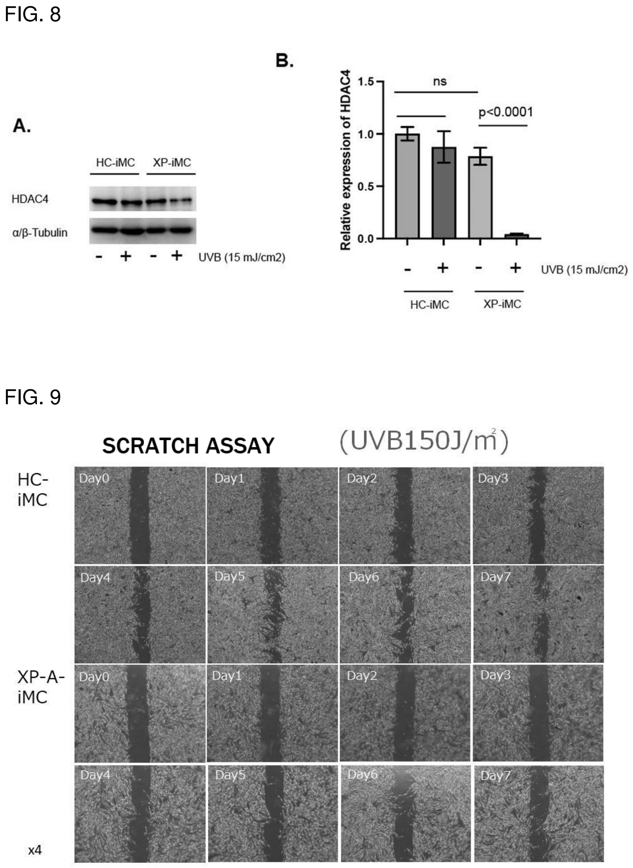

In this Example, histone deacetylase 4 (HDAC4) was identified as a gene strongly showing characteristic behavior out of the 132 gene probes whose expression specifically reduced in the XP-A-iMCs (FIG. 7). It has been known from previous reports that HDAC4 plays an important role in histone modification, is important for cell proliferation, cell cycle progression, differentiation, and development, and is involved in autophagy and mitophagy. Pathology observed in XP can surely be described on the basis of HDAC4, and hence the following possibility is conceivable: HDAC4 is a gene that epigenetically controls a melanocyte. With regard to the behavior of HDAC4, its expression reduced in all MCs 4 hours after their irradiation with UV-B, and recovered in each of the NHEMs and the HC-iMCs 12 hours thereafter, but the expression of HDAC4 tended to fail to recover in the XP-A-iMCs, and continued to reduce 12 hours after the UV irradiation (FIG. 7). The expression of HDAC4 in the XP-A-iMCs was multilaterally recognized by using a RT-PCR method and a western blotting method (FIG. 8A and FIG. 8B).

### (Example 5) Examination in Cell Migration Abilities of XP-A-iMCs

In this Example, the cell migration abilities of the XP-A-iMCs after their UV irradiation were examined by using a scratch assay and digital holographic microscopy.

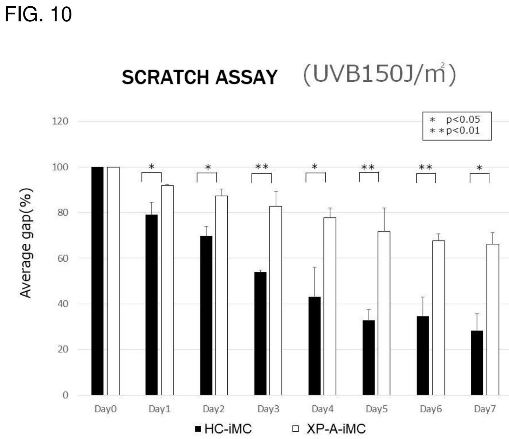

The fact that the movement abilities of the XP-A-iMCs reduced was elucidated by analysis using the scratch assay and the digital holographic microscopy. Specifically, the XP-A-iMCs and the HC-iMCs were seeded into 60-millimeter dishes, and were irradiated with high-dose (150 J/m²) UV-B when the XP-A-iMCs and the HC-iMCs became confluent. After that, fissures were produced by scratching the XP-A-iMCs and the HC-iMCs with a small scraper. After that, the manner in which the fissures were filled up by cell migration was daily observed with a phase-contrast microscope (FIG. 9). The results of the examination of the cell migration abilities by the scratch assay are shown in FIG. 10. It was revealed that in the XP-A-iMCs in which HDAC4 reduced, the cell migration was significantly hindered as compared to the HC-iMCs.

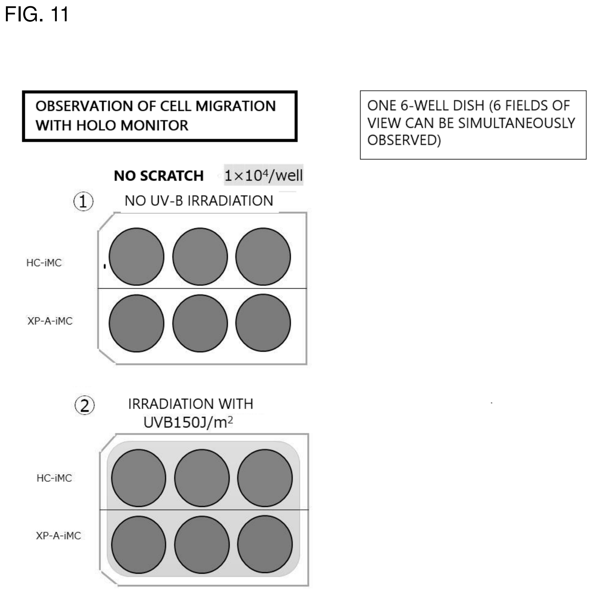

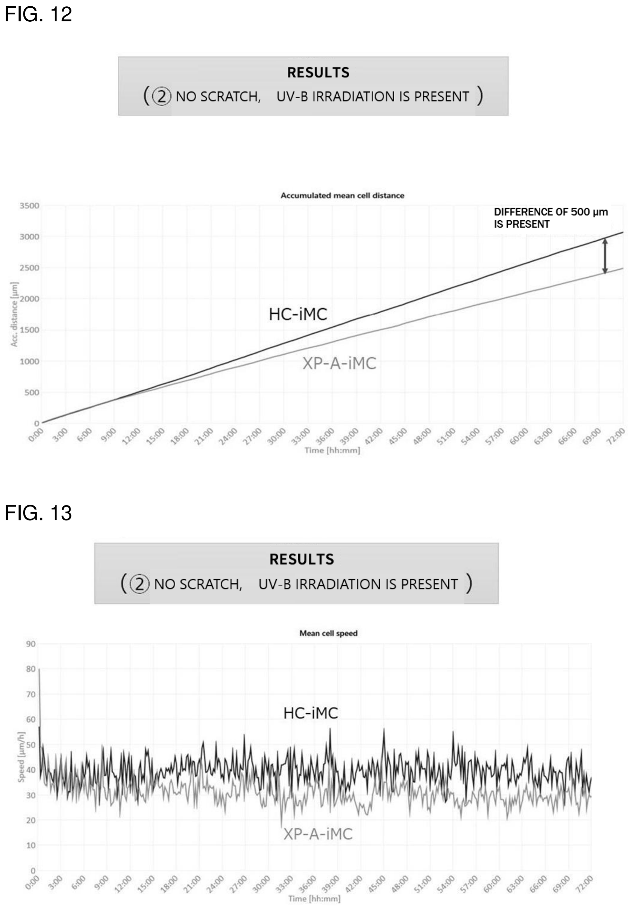

The cell migration was also examined with a digital holographic microscope (FIG. 11). A HoloMonitor^{™} M4 microscope (Phase Holographic Imaging AB) was used in the imaging and tracking of the movements of the XP-A-iMCs and the HC-iMCs after their irradiation with high-dose (150 J/m²) UV-B. The XP-A-iMCs and the HC-iMCs (1×10⁴ melanocytes) were seeded into a 6-well plate, and were irradiated with the high-dose (150 J/m²) UV-B, followed by the tracking of their migration 72 hours after the irradiation. The distance by which the cells migrated is shown in FIG. 12, and the speed of the cell migration is shown in FIG. 13. It was able to be recognized that in the XP-A-iMCs after the irradiation with the UV-B in which HDAC4 reduced, the migration abilities reduced. The foregoing suggested a possibility that the phenotype of the XP-A-iMCs caused pigmentation or depigmentation, which was a skin symptom characteristic of an XP-A patient. In addition, in Example 2, it was shown that the survival rate of the XP-A-iMCs was reduced by photoirradiation. Accordingly, there is a possibility that it can be expected that a substance that alleviates the cell injuries of melanocytes is obtained by: adding a candidate substance to the culture system of the melanocytes; and evaluating the survival rate of the melanocytes and the cell migration abilities of the melanocytes.

### (Example 6) Examination in Cellular Senescence of XP-A-iMCs

In this Example, whether or not the cellular senescence of the XP-A-iMCs after their UV irradiation occurred was examined by using an immunofluorescent antibody method.

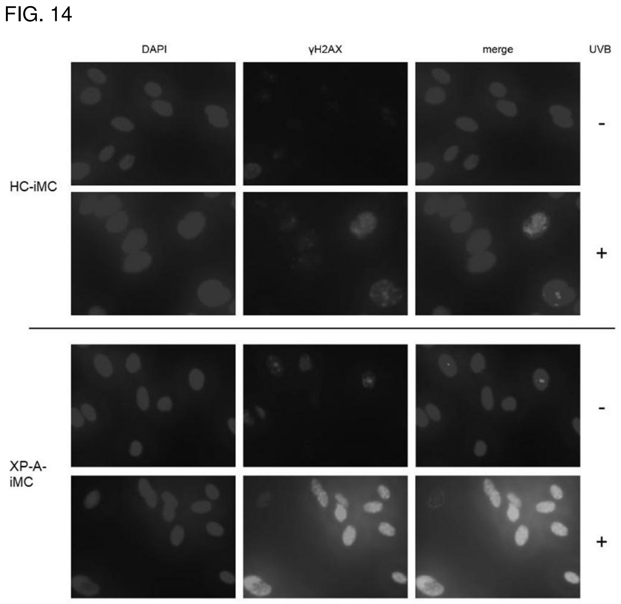

The XP-A-iMCs and the HC-iMCs were seeded into 60-millimeter dishes, and were irradiated with high-dose (150 J/m²) UV-B when the XP-A-iMCs and the HC-iMCs became about 70% confluent. 24 Hours after that, the expression of γH2AX, which was an indicator of DNA damage and was also an indicator for the evaluation of cellular senescence, was examined. In the XP-A-iMCs, a marked increase in expression of γH2AX as compared to that in the HC-iMCs was recognized by a fluorescent antibody method, and hence it was revealed that the cellular senescence was promoted (FIG. 14).

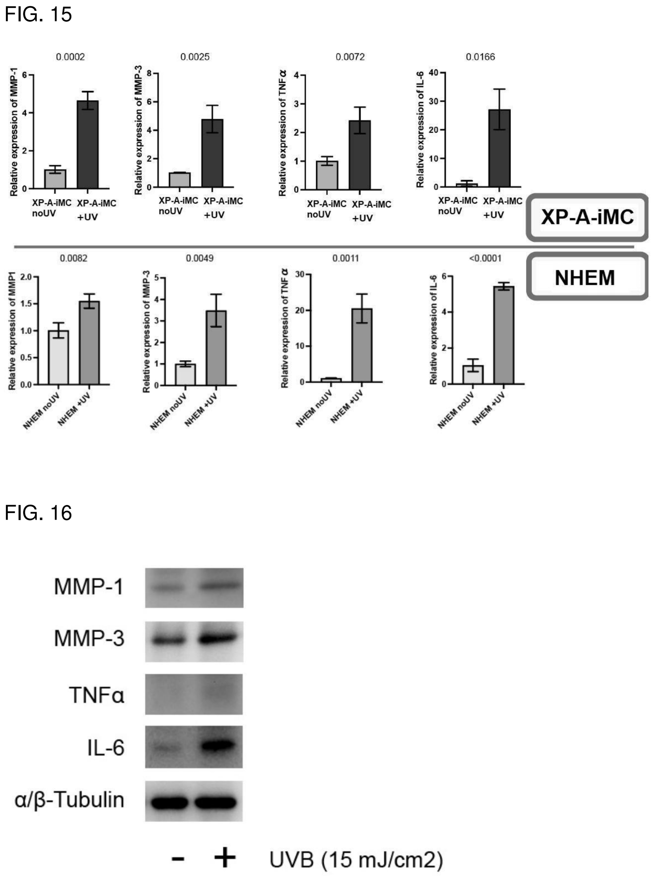

Further, the inventors have conceived that the melanocytes that have caused the cellular senescence change a senescence-associated secretory phenotype (SASP) factor group, which causes inflammation, and have examined an increase in expression of each of many SASP factor groups through use of a RT-PCR method. In the XP-A-iMCs in which the expression of HDAC4 reduced after the irradiation with the UV light (150 J/m²), an increase in expression of each of many SASP factor groups was recognized (FIG. 15). Further, the expression of the SASP factor group was recognized by a western blotting method (FIG. 16). It has been reported in other cancer types that the SASP factor group whose expression has increased causes chronic inflammation. However, in an XP-A patient, the fact that the SASP factor group whose expression has increased causes chronic inflammation may be a cause for a skin symptom, which is marked promotion of photoaging including an increase in occurrence of characteristic photocarcinogenesis. In view of the foregoing, it was suggested that the suppression of a reduction in expression of HDAC4 enabled comprehensive suppression of the skin symptoms of XP. Meanwhile, even in the NHEMs, in the case where remarkably high-dose (500 J/m²) UV-B was applied thereto, an increase in expression of the SASP factor group was recognized as in the case where the XP-A-iMCs were irradiated with the UV light (150 J/m²) (FIG. 15).

### (Example 7) Reduction in Expression of HDAC4 in NHEMs

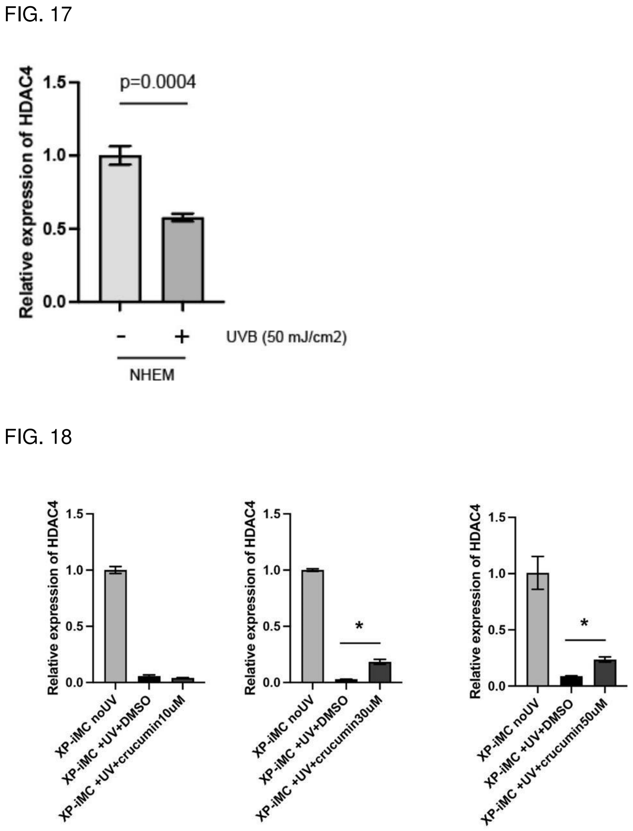

In this Example, the expression of HDAC4 in the NHEMs was recognized. The NHEMs were seeded into a 60-millimeter dish, and were irradiated with remarkably high-dose (500 J/m²) UV-B. 24 Hours after that, the expression of HDAC4 was recognized by using a RT-PCR method (FIG. 17). It was able to be recognized that pathology in which the expression of HDAC4 was markedly reduced by UV irradiation was also applicable to a melanocyte from a healthy person when the amount of UV light was remarkably increased. Thus, a possibility that a reduction in expression of HDAC4 was generally important in photoaging was suggested.

### (Example 8) Improving Action on Expression of HDAC4 by Curcumin

The XP-A-iMCs were seeded into a 60-millimeter dish, and were irradiated with high-dose (150 J/m²) UV-B when the XP-A-iMCs became about 70% confluent. After that, curcumin was added to a cell medium, and the XP-A-iMCs were cultured for 24 hours. After that, the expression of HDAC4 was recognized by using a RT-PCR method. Dimethyl sulfoxide (DMSO) serving as a solvent for curcumin was used as a control. It was recognized that the expression of HDAC4 reduced by the irradiation with the high-dose (150 J/m²) UV-B significantly recovered (FIG. 18). In addition, it was able to be recognized that the effect was dependent on the concentration of curcumin.

### (Example 9) Improving Action on Expression of HDAC4 by C-646

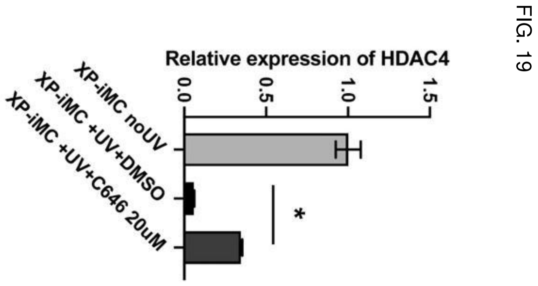

The XP-A-iMCs were seeded into a 60-millimeter dish, and were irradiated with high-dose (150 J/m²) UV-B when the XP-A-iMCs became about 70% confluent. After that, C-646 was added to a cell medium, and the XP-A-iMCs were cultured for 24 hours. After that, the expression of HDAC4 was recognized by using a RT-PCR method (FIG. 19). Dimethyl sulfoxide (DMSO) serving as a solvent for C-646 was used as a control. It was recognized that the expression of HDAC4 reduced by the irradiation with the high-dose (150 J/m²) UV-B significantly recovered.

### Industrial Applicability

The prophylactic and/or therapeutic agent for a skin disorder associated with a histone deacetylase 4 abnormality of the present invention, the agent including the histone acetyltransferase inhibitor as an active ingredient, is very useful because the agent can prevent and/or treat the skin disorder associated with the histone deacetylase 4 abnormality, and is hence applicable not only to an XP patient but also to many skin diseases due to, for example, photoaging. The agent is useful particularly for the XP patient because no effective therapeutic method for XP has been established. In addition, the screening of a novel prophylactic and/or therapeutic agent for a skin disorder paying attention to HDAC4 and a melanocyte can be performed.

## Claims

1. A prophylactic and/or therapeutic agent for a skin disorder associated with a histone deacetylase 4 abnormality, comprising a histone acetyltransferase inhibitor as an active ingredient.

2. The prophylactic and/or therapeutic agent for a skin disorder according to claim 1, wherein the skin disorder is a skin disorder associated with a histone deacetylase 4 abnormality in a melanocyte caused by light exposure.

3. The prophylactic and/or therapeutic agent for a skin disorder according to claim 1, wherein the skin disorder is a skin disorder due to photoaging.

4. The prophylactic and/or therapeutic agent for a skin disorder according to claim 1, wherein the skin disorder is a skin disorder due to xeroderma pigmentosum.

5. The prophylactic and/or therapeutic agent for a skin disorder according to claim 1, wherein the prophylactic and/or therapeutic agent for a skin disorder associated with a histone deacetylase 4 abnormality has an alleviating action on an expression abnormality of histone deacetylase 4.

6. The prophylactic and/or therapeutic agent for a skin disorder according to claim 1, wherein the histone acetyltransferase inhibitor is one or a plurality of kinds selected from the group consisting of: curcumin; C-646; A-485; anacardic acid; butyrolactone; CTPB; and garcinol.

7. A method of screening a prophylactic and/or therapeutic agent for a skin disorder, comprising adding a candidate substance to a culture system of a melanocyte to sort out a substance that alleviates a cell injury of the melanocyte.

8. The screening method according to claim 7, wherein the substance that alleviates the cell injury of the melanocyte is obtained by detecting histone deacetylase 4 and/or a senescence-associated secretory phenotype factor.

9. The screening method according to claim 7, wherein the substance that alleviates the cell injury of the melanocyte is obtained by evaluating a survival rate of the melanocyte.

10. The screening method according to claim 7, wherein the substance that alleviates the cell injury of the melanocyte is obtained by evaluating a cell migration ability of the melanocyte.

11. The screening method according to claim 7, wherein the melanocyte is a pluripotent stem cell-derived melanocyte.

12. The screening method according to claim 11, wherein the pluripotent stem cell-derived melanocyte is a pluripotent stem cell-derived melanocyte produced from a pluripotent stem cell derived from a xeroderma pigmentosum patient.

13. The screening method according to claim 7, wherein the cell injury of the melanocyte is a cell injury due to light exposure.

14. The screening method according to claim 13, wherein the cell injury due to the light exposure is a skin disorder associated with a histone deacetylase 4 abnormality.

15. A method of testing a skin disorder, comprising a step of detecting an abnormality of histone deacetylase 4 and/or a senescence-associated secretory phenotype factor from skin collected from a subject.

16. The method of testing a skin disorder according to claim 15, wherein the senescence-associated secretory phenotype factor is one or a plurality of kinds selected from MMP-1, MMP-3, TNFα, and IL-6.

17. The method of testing a skin disorder according to claim 15, wherein the detecting the abnormality of histone deacetylase 4 and/or the senescence-associated secretory phenotype factor is performed by using the following (1) or (2):
(1) a nucleic acid probe or a nucleic acid primer capable of specifically detecting a transcription product of a histone deacetylase 4 gene and/or a senescence-associated secretory phenotype factor gene; and
(2) an antibody that specifically recognizes histone deacetylase 4 and/or the senescence-associated secretory phenotype factor.

18. A kit for testing a skin disorder, comprising a substance capable of detecting an abnormality of histone deacetylase 4 and/or a senescence-associated secretory phenotype factor.
